# EUROPEAN PATENT APPLICATION

(11) **EP 1 184 463 A1**
(43) Date of publication of application: **06.03.2002**
(21) Application number: 01912323.1
(22) Date of filing: 13.03.2001
(51) Int. Cl.: C12N 15/79, G01N 33/68, C12P 21/02

(54) **METHOD OF DETECTING PROTEIN-PROTEIN INTERACTION**

(30) Priority: 15.03.2000 JP 2000073095; 24.08.2000 JP 2000254418
(71) Applicant: Japan Science and Technology Corporation, Kawaguchi-shi, Saitama 332-0012 (JP)
(72) Inventor: KATO, Seishi, Sagamihara-shi, Kanagawa 229-0014 (JP); EGUCHI, Chikashi, 304, CLL Excellence Asamizodai I, Sagamihara-shi, Kanagawa 228-0828 (JP); NAGATA, Naoki, Sagamihara-shi, Kanagawa 229-0012 (JP); OTAKE, Miyako, Tokyo 210-0012 (JP)
(74) Representative: Gardner, Rebecca
(86) International application number: JP0101973
(87) International publication number: WO0168885

(57) **Abstract**

The invention provides a detection method of protein-protein interaction that detects the interaction of a protein X with a protein Y having a reporter function in a eucaryotic cell, which comprises modifying the protein X and/or the protein Y to be expressed with different localization patterns in the cell, co-expressing the protein X and the protein Y in the eucaryotic cell, and identifying the intracellular localization of the protein Y by its reporter function that serves as the index thereof. The invention also provides a method for detecting the inhibitory or promotive action of a compound Z on the interaction of the proteins X and Y in a eucaryotic cell.

## Description

### Technical Field

The invention of this application relates to a detection method of protein-protein interaction. According to the method, it is possible to screen intracellular proteins to find out a specific protein that interacts with a target protein under intracellular condition. Therefore, the method is a promising means for clarifying the protein network in cells. Clarifying the protein network in cells is useful for developing new medicines. According to the method, it is possible to screen low-molecular substances for new medicines.

### Background Art

The whole base sequence analysis of the genome in a cell is clarifying the primary structure of every protein constituting the cell. The next theme in the art is to analyze and clarify what kind of intracellular network is formed by the proteins in a cell and what kind of life activity of the cell is born by the proteins therein. The protein network in a cell, if clarified, is helpful in clarification of the molecular mechanism of diseases to be caused by protein disorders, and in developing the medicines for the diseases.

For the protein network clarification, it is necessary to clarify the protein-protein interaction in a cell as to which type of intracellular protein interacts with which other type thereof. Various methods for detecting the protein-protein interaction in a cell have heretofore been developed, including, for example, (1) affinity chromatography, (2) affinity blotting, (3) immunoprecipitation, (4) crosslinking, (5) mass spectrometry, (6) surface plasmon resonance, (7) yeast two-hybrid transcription activation, (8) two-hybrid reconstruction, and (9) fluorescence resonance energy transfer, which, however, all have some problems (E. M. Phizicky and S. Fields, Microbiol. Rev., 59: 94-123, 1995; A. R. Mendelsohn and R. Brent, Science, 284: 1948-1950, 1999). Of those, the methods (1) to (6) require protein production and purification that needs much labor. In addition, all these are for in-vitro interaction, and therefore do not always reflect the corresponding intracellular interaction. As the case may be, they are often for artificial binding. On the other hand, the other methods (7) to (9) are for detecting the reconstruction of reporter proteins or the approach of the two to each other on the basis of the transcription activity or the enzymatic activity of the reporter proteins or on the basis of the fluorescence resonance energy transfer between the reporter proteins, that results from the interaction of target proteins in a cell through intracellular expression of the cDNAs that separately code for the two interacting target proteins, to give a fused gene of each cDNA and a reporter cDNA for it.. For effective use of the methods (7) to (9), however, the two reporter proteins each separately fused to the two target proteins must be bound to each other by the binding of the target proteins, or they must be near to each other within a predetermined distance. Therefore, the methods are not applicable to target proteins which are too large or which do not have a flexible structure. The method (7) is used most frequently as its limitations on the protein structure are relatively small as compared with those of the other methods. However, the method (7) is defective in that the yeast growth therein is slow and the efficiency of transformation therein is low. When the target proteins themselves have a transcriptional activity, this system cannot be used for them.

The invention of this application has been made in consideration of the prior art problems noted above, and its object is to provide a method for surely detecting the protein-protein interaction in a cell in a simplified manner.

Another object of the invention of this application is to provide a method for detecting the action of a compound on the protein-protein interaction in a cell.

### Disclosure of The Invention

For the first aspect of the invention to solve the above-mentioned problems, the present application provides a method for detecting the interaction of a protein X with a protein Y having a reporter function in a eucaryotic cell, which comprises;
(1) modifying the protein X and/or the protein Y to be expressed with different localization patterns in the cell,
(2) co-expressing the protein X and the protein Y in the eucaryotic cell, and
(3) identifying the intracellular localization of the protein Y by its reporter function that serves as the index thereof.

For the second aspect of the invention, the present application provides a method for detecting the inhibitory or promotive action of a compound Z on the interaction of a protein X with a protein Y having a reporter function in a eucaryotic cell, which comprises;
(1) modifying the protein X and/or the protein Y to be expressed with different localization patterns in the cell,
(2) co-expressing the protein X and the protein Y in the eucaryotic cell,
(3) introducing the compound Z into the eucaryotic cell, and
(4) identifying the intracellular localization of the protein Y by its reporter function that serves as the index thereof.

In the first and second aspects of the invention, one preferred embodiment is that the protein Y having a reporter function is a fusion protein Y-B with a reporter protein B, and another preferred embodiment is that the reporter protein B is a fluorescent protein.

In the first and second aspects of the invention, still another preferred embodiment is that, in the step (1), the intracellular localization signal sequence of the protein X and/or the protein Y is mutated, or the protein X is fused with a protein A of which the intracellular localization signal sequence differs from that of the protein Y.

In the second aspects of the invention, still another preferred embodiment is that the compound Z is, for example, a low-molecular compound, a peptide or a protein, and in case where the compound Z is a low-molecular compound, it coexists with the eucaryotic cell, and in case where the compound Z is a protein or a peptide, it is expressed in the eucaryotic cell.

In the first and second aspects of the invention, the proteins X, Y, A and B may be matured proteins, parts thereof, or peptides. In these, the fusion protein X-A and the fusion protein Y-B may be a fusion protein A-X and a fusion protein B-Y, respectively, in which "-" indicates a peptide bond.

### Brief Description of Drawings

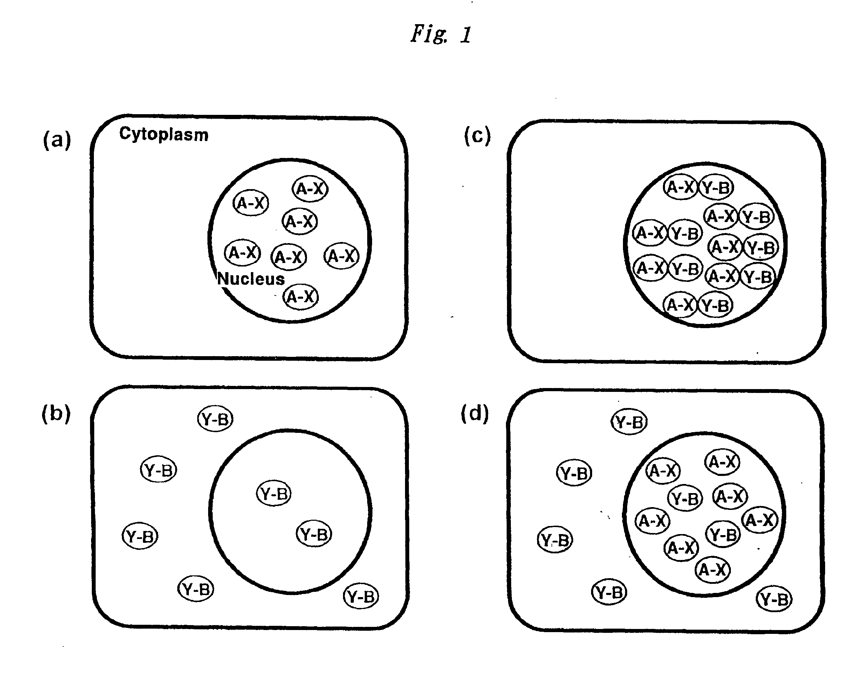

Fig. 1 is a schematic view showing the principle of the first invention. (a) indicates the distribution of A-X expressed alone; (b) indicates the distribution of Y-B expressed alone; (c) indicates the distribution of A-X and Y-B co-expressed together where X and Y have the ability to bind to each other; and (d) indicates the distribution of A-X and Y-B co-expressed together where X and Y do not have the ability to bind to each other.

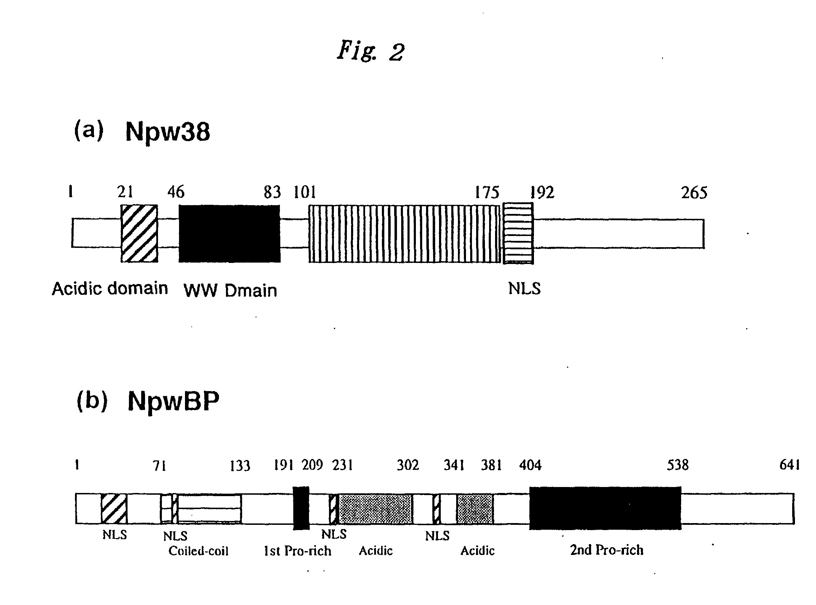

Fig. 2 is a view showing the domain structures of Npw38 (a) and NpwBP(b).

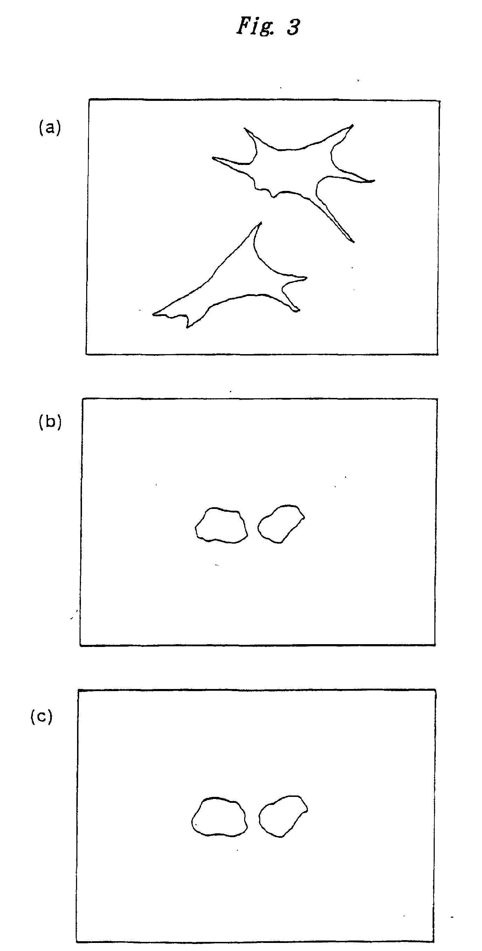

Fig. 3 is a view drawn from microscopic pictures of protein expression. (a) indicates the localization pattern of the fusion protein NpwBP(P2)-GFP; (b) indicates the localization pattern of NpwBP(P2)-GFP co-expressed with HA-Npw38; and (c) indicates the localization pattern of HA-Npw38.

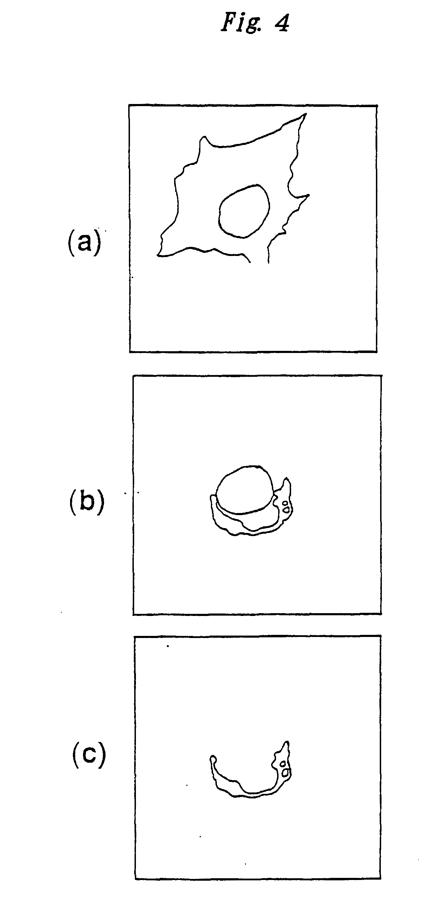

Fig. 4 is a view drawn from microscopic pictures of protein expression. (a) indicates the localization pattern of a fusion protein NpwBP(ΔC)-GFP; (b) indicates the localization pattern of NpwBP(ΔC)-GFP co-expressed with NpwBP(P2)-Bcl-X_{L}; and (c) indicates a picture of an antibody-stained image of mitochondria.

### Best Mode for Carrying Out The Invention

One best embodiment for carrying out the first invention is described. In this embodiment, the protein Y used is a fusion protein Y-B with a reporter B (protein or peptide). Concretely, an expression vector for expressing a fusion protein Y-B with a reporter B of being fused to the N-terminal or C-terminal of Y is prepared, and this is used together with an expression vector for a fusion protein X-A in the manner described below.

### Step 1:

Preparing an expression vector for expressing the fusion protein X-A with a protein A or a peptide A of being fused to the N-terminal or the C-terminal of X, in which the intracellular localization signal sequence of the protein A or peptide A differs from that of the protein Y.

### Step 2:

The Y-B expression vector previously prepared is introduced into a eucaryotic cell together with the X-A expression vector prepared in the step 1. For control, a cell with only the Y-B expression vector introduced thereinto is also prepared.

### Step 3:

The cells are cultured, and the site in these in which the reporter B is localized is detected by any optical means or biochemical means to thereby judge the presence or absence of the Y-B localization site difference between the cell with only the Y-B expression vector introduced thereinto and the cell with the two expression vectors introduced thereinto. In this step, when the intracellular localization of Y-B is identical with that of X-A, then it means that X and Y bind to each other.

Fig. 1 is a schematic view showing the principle of the first aspect of the invention, concretely illustrating one example in which a protein A having an intranuclear localization signal sequence is used and this is fused to form a fusion protein A-X. Depending on the intranuclear localization signal of A, the fusion protein A-X is localized in the nucleus of the cell (Fig. 1a). On the other hand, since a fusion protein Y-B does not have the localization signal which A has, it exists in a site of the cell in which A-X does not exist (in this example, Y-B exists everywhere in the cell including its cytoplasm and nucleus) (Fig. 1b). When A-X and Y-B are both expressed in the cell and when X and Y have the ability to bind to each other, then Y-B binds to A-X via their binding sites, and the two are led into the nucleus of the cell by the intranuclear localization signal which A has (Fig. 1c). On the other hand, when X and Y do not have the ability to bind to each other, then A-X is led into the nucleus, but the distribution of Y-B does not change (Fig. 1d). Accordingly, when the distribution of Y-B in the cell in which Y-B only has been expressed differs from the distribution of Y-B in the cell in which both A-X and Y-B have been expressed, then it is judged that X binds to Y, based on the reporter B that serves as the index of intracellular protein distribution difference. For this, the sameness, if any, between the localization of Y-B and the localization of A-X (this may be presumed from the type of the localization signal of A) may also be a factor for the judgment.

In case where X has a localization signal sequence, it does not always require X to form such a fusion protein X-A. In this case, the localization signal sequence that X originally has may be deleted or mutated, and A may be fused to the resulting X. A may be a protein having a localization signal sequence or may also be a peptide containing a minimal unit that serves as a localization signal. The localization signal includes, for example, a nuclear localization signal, a mitochondrial localization signal, a Golgi localization signal, and a membrane localization signal. Of those, preferred are a nuclear localization signal and a mitochondrial signal that facilitate the identification of the intended localized sites. In addition to such a localization signal sequence, A may contain a site having a reporter function. Its reporter function, if used, makes it possible to confirm the site where the fusion protein X-A is localized. Needless-to-say, since the site where the fusion protein X-A is localized can be presumed with accuracy, depending on the type of the localization signal of A, the reporter function of A is not indispensable.

In case where Y has a reporter function, it does not always require the reporter B to form such a fused protein Y-B. In this case, when the localization signal of Y is the same as that of X, it is deleted or varied. The reporter B includes, for example, green fluorescent protein (GFP), reciferase, β-galactosidase, chloramphenicol acetyltransferase, and various epitope tags. For it, preferred are fluorescent proteins such as GFP, as facilitating the detection of the intended protein localization through microscopic cell observation.

For expressing the fusion proteins in a cell, employable is a method that comprises preparing fusion protein expression vectors followed by introducing the two types of vectors into the cell. However, in case where a cell to produce the protein X that is localized in a specific site are used, only the Y-B expression vector may be introduced into the cell.

For the fusion protein expression vector, employable are all types of expression vectors for eucaryotic cell so far as they have a promoter, a splicing region and/or a poly(A) additional site. For example, they include pKA1, pCDM8, pSVK3, pMSG, pSVL, pBK-CMV, pIND/V5-His, pFLAG-CMV-2, pEGFP-N1, pEGFP-C1, pBK-RSV, EBV vector, pRS, and pYES2. With any of these vectors, cDNA or DNA fragment each encoding any of X, Y, A and B are cloned to prepare the intended, fusion protein X-A or Y-B expression vectors. These cDNAs and DNA fragments may be prepared, for example, by fixing any one of X and Y while cloning the other in the DNA fragments derived from a cDNA library or a random DNA library, whereby the DNA fragment that encodes the protein capable of binding to the fixed protein can be screened from the library.

The cell to express the fusion proteins may be all types of eucaryotic cells, including, for example, cultured mammal cells such as monkey kidney cells COS7, Chinese hamster ovarian cells CHO, various human tumor cell lines; and also budding yeasts, fission yeasts, silkworm cells, Xenopus laevis cells. For introducing the expression vector into eucaryotic cell, employable is any known method such as an electroporation method, a calcium phosphate method, a liposome method or a DEAE-dextran method.

For detecting the reporter in the cell containing it, employable are various methods depending on the type of the reporter. In case where a fluorescent protein such as GFP is used for the reporter, the cell containing it may be observed with a fluorescence microscope to confirm the site in which the reporter is localized. In case where an enzyme is used for the reporter, the cell containing it is fixed and then a substrate is added thereto to lead to enzymatic reaction therein. With that, the site in the cell in which the reaction product exists is confirmed through microscopic observation. Alternatively, the cell containing the reporter protein is homogenized, and then the intracellular organ that contains the reporter protein is detected through enzymatic reaction to thereby determine the site in which the reporter protein is localized. In case where an epitope tag is used for the reporter, the cell containing it is immunostained with an antibody against the reporter and the site in which the reporter is localized is confirmed through fluorescence microscopy.

The second aspect of the invention of this application is a screening method for a compound Z that inhibits or promotes the interaction of X and Y. In case where Z is a low-molecular compound, it is added to the cell culture in which both the fusion protein X-A and the fusion protein Y-B have been co-expressed, and the localization change of Y-B in the cell is determined based on the reporter B that serves as the index thereof. With that, the influence of Z on the binding of X to Y can be determined. In case where X is a peptide or a protein, a Z expression vector is introduced into cell together with X-A and Y-B expression vectors, and the influence of Z on the localization change of Y-B in the cell is checked.

### Examples

The invention is described in more detail and concretely with reference to the following Examples, which, however, are not intended to limit the scope of the invention of this application.

The following is to demonstrate the detection of the binding of two types of nuclear proteins Npw38 (A. Komuro et al., Nucl. Acids Res., 27: 1957-1965, 1999) and NpwBP (A. Komuro et al., J. Biol. Chem., 274: 36513-36519, 1999). Npw38 (SEQ ID NO: 1) contains a nuclear localization signal sequence (from 176th arginine to 192th lysine), and it is known that the protein is localized in nucleus (Fig. 2a). NpwBP (SEQ ID NO: 2) is a protein that binds to Npw38. It is known that the two bind to each other at the WW domain of Npw38 (from 52nd tryptophan to 78th proline in SEQ ID NO: 1) and the proline-rich region of NpwBP (from 191st proline to 209th proline and from 393rd proline to 538th proline in SEQ ID NO: 2).

### (1) Preparation of Fused Protein Expression Vectors:

An EcoRI-Notl fragment containing a cDNA of GFP, which had been prepared from pEGFP-N1 (from Clontech), was inserted into pKA1 (Kato et al., Gene, 150: 243-250, 1994) at its EcoRI-NotI site to prepare pKA1-GFP. P1 oligomer 1 (SEQ ID NO: 3) and P1 oligomer 2 (SEQ ID NO: 4) were hybridized, then inserted into pKA1-GFP at its EcoRI and Smal to prepare pKA1-NpwBP(P1)-GFP. On the other hand, two primers, P2 primer 1 (SEQ ID NO: 5) and P2 primer 2 (SEQ ID NO: 6) were used to prepare a PCR product, which was then digested with EcoRI and SmaI, and inserted into pKA1-GFP at its EcoRI and SmaI to prepare pKA1-NpwBP(P2)-GFP.

The pKA1-Npw38 (A. Komuro et al., Nucl. Acids Res., 27:1957-1965, 1999) serving as a template was used along with two primers, primer 1 (SEQ ID NO: 7) and primer 2 (SEQ ID NO: 8) to prepare a PCR product. This was digested with EcoRI and SmaI, and then inserted into pKA1-GFP at its EcoRI and Smal to prepare pKA1-Npw38(ΔC)-GFP.

### (2) Fusion Protein Expression in COS7, Immunostaining, and Fluorescence Microscopy:

Monkey kidney-derived, cultured cell COS7 was incubated in a 10% fetal calf serum-containing Dulbecco's modified Eagle's medium (DMEM) in the presence of 5 % CO₂ at 37°C. COS7 cells of 1 × 10⁵ were placed in a 6-well plate (from Nunk, having a well diameter of 3 cm), and further incubated therein in the presence of 5 % CO₂ at 37°C for 22 hours. After the medium was removed, the cell surfaces were washed with a phosphate buffer solution (PBS), and then further washed with a 50 mM Tris-HCl (pH 7.5)-containing DMEM. To these cells, added was a suspension comprising 1 µl of a plasmid suspension, 0.6 ml of DMEM and 3 µl of TRANSFECTAM™ (from IBF), and incubated in the presence of 5 % CO₂ at 37°C for 12 hours. Then, the cells were washed with PBS, and fixed with 4 % paraformaldehyde-containing PBS at room temperature for 10 minutes. The thus-fixed cells were washed with PBS, then treated with 0.2 % Triton X-100, and thereafter blocked with 5 % milk PBS. This was reacted with an anti-HA antibody for 45 minutes, then washed with PBS, and reacted with a rhodamine-binding secondary antibody for 45 minutes. With a fluorescence microscope, the GFP-derived green fluorescence distribution and the antibody-derived red fluorescence distribution were observed to determine the sites in which the respective proteins were localized.

### (3) Npw38-NpwBP Binding Detection (1):

Fusion protein HA-Npw38 (A. Komuro et al., J. Biol. Chem., 274: 36513-36519, 1999) was used. This is a fusion protein A-X in which X is Npw38 and A is HA tag. Since Npw38 contains a inuclear localization signal, A does not require a localization signal. Also used were fusion proteins NpwBP(P1)-GFP and NpwBP(P2)-GFP. These are fusion proteins Y-B in which Y is a proline-rich region of NpwBP and B is GFP. In these, P1 contains amino acid residues of from 190th valine to 210th glutamine in SEQ ID NO: 2; and P2 contains amino acid residues of from 401st glutamine to 541st isoleucine in Seq ID NO: 2.

First, pKA1-NpwBP(P1)-GFP or pKA1-NpwBP(P2)-GFP was singly introduced into COS7 cells, and the localization of these fusion proteins in the cells was checked through fluorescence microscopy based on the green fluorescence of GFP serving as the index thereof. In the two cases, the entire cells each including its cytoplasm and nucleus were found to gave the green fluorescence (Fig. 3a). Next, along with these NpwBP-GFP fusion protein expression vectors, an Npw38 expression vector, pKA1-Npw38 was introduced into COS7 cells, and the green fluorescence was localized in the nucleus of each cell (Fig. 3b). In this stage, the cells were immunostained with an antibody against the tag to check the localization of the fusion protein HA-Npw38. This verified the localization of the fusion protein in the nucleus of each cell (Fig. 3c). On the other hand, when Npw38(W52A) (A. Komuro et al., Nucl. Acids Res., 27: 1957-1965, 1999), which is a WW domain variant of Npw38, was expressed in the cells in place of HA-Npw38, or when GFP was expressed in the cells in place of NpwBP-GFP, no intranuclear localization of the green fluorescence was found. These results confirm that the intranuclear localization of the green fluorescence resulted from the binding of the fusion protein NpwBP-GFP to Npw38 via the WW domain of Npw38.

### (4) Npw38-NpwBP Binding Detection (2):

Systems contrary to those in the above (3) were tried. Concretely, NpwBP was used as a fusion protein X-A. Also in this case, since NpwBP contains a nuclear localization signal, the protein X does not require the additional protein A. On the other hand, for Y-B, used was a fusion protein Npw38(ΔC)-GFP in which the nuclear localization signal in Npw38 for Y was deleted, and B is GFP. The fusion protein Npw38(ΔC)-GFP was expressed in COS7 cells, and the green fluorescence was found to appear in the entire cells each including its cytoplasm and nucleus. In this system, when NpwBP was co-expressed, the green fluorescence was found to move in the nucleus of each cell.

### (5) Detection of Binding-Inhibitory Action:

In the system of the above (2), NpwBP of more than NpwBP(P2)-GFP was co-expressed in the cells along with Npw38 and NpwBP(P2)-GFP. In this case, the intranuclear localization of NpwBP(P2)-GFP was inhibited. This will be because the over-expressed NpwBP bound to Npw38 to inhibit the binding of Npw38 to NpwBP(P2)-GFP, and therefore NpwBP(P2)-GFP could not move to the nuclei of the cells. As in this, when the binding-inhibitory substance is present in the above-mentioned system, it inhibits the localization of the GFP-fused protein. Taking advantage of this system, therefore, the binding-inhibitory factor can be searched out.

### (6) Application of Mitochondrial Localization Signal:

For the fusion protein X-A, herein used was NpwBP(P2)-Bcl-X_{L} in which X is NpwBP(P2), and A is Bcl-X_{L}, a type of mitochondrial protein. First, a full-length cDNA clone HPO3564 of Bcl-X_{L} (its ORF is in SEQ ID NO: 9) was selected from a human full-length cDNA bank (Kato et al., Gene, 150: 243-250, 1994). Using a Bcl primer P1 (SEQ ID NO: 10) and another Bcl primer P2 (SEQ ID NO: 11), this was processed to produce its PCR product, which was then digested with Smal and NotI and inserted into pKA1-GFP at its Smal and NotI to construct pKA1-Bcl. Next, in the same manner as above, the full-length cDNA clone HP03564 of Bcl-X_{L} was used as a template, along with the Bcl primer P1 (SEQ ID NO: 10) and another Bcl primer P3 (SEQ ID NO:12) to prepare a PCR product. This was digested with Smal and PmaCI, and inserted into pKA1-Bcl at its SmaI to construct pKA1-Bc1-SmaI. pKA1-NpwBP(P2)-GFP was digested with EcoRI and Smal, and then a fragment encoding NpwBP(P2) was inserted into pKA1-Bc1-Smal at its EcoRI and Smal to construct pKA1-NpwBP(P2)-Bcl-X_{L}.

When the fusion protein Npw38(ΔC)-GFP was expressed alone in the COS7 cells, the green fluorescence was found to appear in the cytoplasm and everywhere in each cell, as in the above (4). However, when the fusion protein NpwBP(P2)-Bcl-X_{L} was co-expressed therein, the mitochondrial region of each cell also gave the fluorescence (Fig. 4). The mitochondrial region in each cell was detected through immunostaining with an anti-human mitochondrial antibody (Leinco Technologies, Inc.). The above-mentioned results confirm that Npw38(ΔC)-GFP bound to NpwBP(P2)-Bcl-X_{L} was localized also in the mitochondrial region of each cell. This means that mitochondrial localization signals are employable in the system of the invention.

### Industrial Applicability

As described in detail hereinabove, the invention of this application provides a method of rapidly detecting any desired protein-protein interaction in an intracellular condition, not requiring a process of protein production and purification. According to the method of the invention, the interaction of different types of proteins in a cell can be detected, and the intracellular protein network in the cell can be clarified. Based on the information of intracellular protein-protein interaction thus obtained according to the invention, novel medicines can be created, and diseases can be clarified on the molecular level.

## Claims

1. A detection method of protein-protein interaction that detects the interaction of a protein X with a protein Y having a reporter function in a eucaryotic cell, which comprises;
(1) modifying the protein X and/or the protein Y to be expressed with different localization patterns in the cell,
(2) co-expressing the protein X and the protein Y in the eucaryotic cell, and
(3) identifying the intracellular localization of the protein Y by its reporter function that serves as the index thereof.

2. The method as claimed in claim 1, wherein the protein Y is a fusion protein Y-B with a reporter protein B.

3. The method as claimed in claim 2, wherein the reporter protein B is a fluorescent protein.

4. The method as claimed in claim 1, wherein, in the step (1), the intracellular localization signal sequence of the protein X and/or the protein Y is mutated.

5. The method as claimed in claim 1, wherein, in the step (1), the protein X is fused with a protein A of which the intracellular localization signal sequence differs from that of the protein Y.

6. A detection method of protein-protein interaction that detects the inhibitory or promotive action of a compound Z on the interaction of a protein X with a protein Y having a reporter function in a eucaryotic cell, which comprises;
(1) modifying the protein X and/or the protein Y to be expressed with different localization patterns in the cell,
(2) co-expressing the protein X and the protein Y in the eucaryotic cell,
(3) introducing the compound Z into the eucaryotic cell, and
(4) identifying the intracellular localization of the protein Y by its reporter function that serves as the index thereof.

7. The method as claimed in claim 6, wherein the protein Y is a fusion protein Y-B with a reporter protein B.

8. The method as claimed in claim 7, wherein the reporter protein B is a fluorescent protein.

9. The method as claimed in claim 6, wherein, in the step (1), the intracellular localization signal sequence of the protein X and/or the protein Y is mutated.

10. The method as claimed in claim 6, wherein, in the step (1), the protein X is fused with a protein A of which the intracellular localization signal sequence differs from that of the protein Y.

11. The method as claimed in claim 6, wherein, in the step (3), the compound Z is coexisted with the eucaryotic cell.

12. The method as claimed in claim 6, wherein, in the step (3), the compound Z is expressed in the eucaryotic cell.


SEQUENCE LISTING

<110> Japan Science and Technology Corporation

<120> A Detection Method of Protein-Protein Interaction

<130> 01-F-01PCT

<140> PCT/JP01/01973
<141> 2001-03-13

<150> JP2000-073095
<151> 2000-03-15
<150> JP2000-254418
<151> 2000-08-24

<160> 12

<170> PatentIn Ver. 2.1

<210> 1
<211> 265
<212> PRT
<213> Homo sapiens

<400> 1

<210> 2
<211> 641
<212> PRT
<213> Homo sapiens

<400> 2
Met Gly Arg Arg Ser Thr Ser Ser Thr Lys Ser Gly Lys Phe Met Asn
1               5                   10                  15
Pro Thr Asp Gln Ala Arg Lys Glu Ala Arg Lys Arg Glu Leu Lys Lys
            20                  25                  30
Asn Lys Lys Gln Arg Met Met Val Arg Ala Ala Val Leu Lys Met Lys
        35                  40                  45
Asp Pro Lys Gln Ile Ile Arg Asp Met Glu Lys Leu Asp Glu Met Glu
    50                  55                  60
Phe Asn Pro Val Gln Gln Pro Gln Leu Asn Glu Lys Val Leu Lys Asp
65                  70                  75                  80
Lys Arg Lys Lys Leu Arg Glu Thr Phe Glu Arg Ile Leu Arg Leu Tyr
                85                  90                  95
Glu Lys Glu Asn Pro Asp Ile Tyr Lys Glu Leu Arg Lys Leu Glu Val
            100                 105                 110
Glu Tyr Glu Gln Lys Arg Ala Gln Leu Ser Gln Tyr Phe Asp Ala Val
        115                 120                 125
Lys Asn Ala Gln His Val Glu Val Glu Ser Ile Pro Leu Pro Asp Met
    130                 135                 140
Pro His Ala Pro Ser Asn Ile Leu Ile Gln Asp Ile Pro Leu Pro Gly
145                 150                 155                 160
Ala Gln Pro Pro Ser Ile Leu Lys Lys Thr Ser Ala Tyr Gly Pro Pro
                165                 170                 175

Thr Arg Ala Val Ser Ile Leu Pro Leu Leu Gly His Gly Val Pro Arg
            180                 185                 190
Leu Pro Pro Gly Arg Lys Pro Pro Gly Pro Pro Pro Gly Pro Pro Pro
        195                 200                 205
Pro Gln Val Val Gln Met Tyr Gly Arg Lys Val Gly Phe Ala Leu Asp
    210                 215                 220
Leu Pro Pro Arg Arg Arg Asp Glu Asp Met Leu Tyr Ser Pro Glu Leu
225                 230                 235                 240
Ala Gln Arg Gly His Asp Asp Asp Val Ser Ser Thr Ser Glu Asp Asp
                245                 250                 255
Gly Tyr Pro Glu Asp Met Asp Gln Asp Lys His Asp Asp Ser Thr Asp
            260                 265                 270
Asp Ser Asp Thr Asp Lys Ser Asp Gly Glu Ser Asp Gly Asp Glu Phe
        275                 280                 285
Val His Arg Asp Asn Gly Glu Arg Asp Asn Asn Glu Glu Lys Lys Ser
    290                 295                 300
Gly Leu Ser Val Arg Phe Ala Asp Met Pro Gly Lys Ser Arg Lys Lys
305                 310                 315                 320
Lys Lys Asn Met Lys Glu Leu Thr Pro Leu Gln Ala Met Met Leu Arg
                325                 330                 335
Met Ala Gly Gln Glu Ile Pro Glu Glu Gly Arg Glu Val Glu Glu Phe
            340                 345                 350
Ser Glu Asp Asp Asp Glu Asp Asp Ser Asp Asp Ser Glu Ala Glu Lys
        355                 360                 365
Gln Ser Gln Lys Gln His Lys Glu Glu Ser His Ser Asp Gly Thr Ser
    370                 375                 380
Thr Ala Ser Ser Gln Gln Gln Ala Pro Pro Gln Ser Val Pro Pro Ser
385                 390                 395                 400
Gln Ile Gln Ala Pro Pro Met Pro Gly Pro Pro Pro Leu Gly Pro Pro
                405                 410                 415

Pro Ala Pro Pro Leu Arg Pro Pro Gly Pro Pro Thr Gly Leu Pro Pro
            420                 425                 430
Gly Pro Pro Pro Gly Ala Pro Pro Phe Leu Arg Pro Pro Gly Met Pro
        435                 440                 445
Gly Leu Arg Gly Pro Leu Pro Arg Leu Leu Pro Pro Gly Pro Pro Pro
    450                 455                 460
Gly Arg Pro Pro Gly Pro Pro Pro Gly Pro Pro Pro Gly Leu Pro Pro
465                 470                 475                 480
Gly Pro Pro Pro Arg Gly Pro Pro Pro Arg Leu Pro Pro Pro Ala Pro
                485                 490                 495
Pro Gly Ile Pro Pro Pro Arg Pro Gly Met Met Arg Pro Pro Leu Val
            500                 505                 510
Pro Pro Leu Gly Pro Ala Pro Pro Gly Leu Phe Pro Pro Ala Pro Leu
        515                 520                 525
Pro Asn Pro Gly Val Leu Ser Ala Pro Pro Asn Leu Ile Gln Arg Pro
    530                 535                 540
Lys Ala Asp Asp Thr Ser Ala Ala Thr Ile Glu Lys Lys Ala Thr Ala
545                 550                 555                 560
Thr Ile Ser Ala Lys Pro Gln Ile Thr Asn Pro Lys Ala Glu Ile Thr
                565                 570                 575
Arg Phe Val Pro Thr Ala Leu Arg Val Arg Arg Glu Asn Lys Gly Ala
            580                 585                 590
Thr Ala Ala Pro Gln Arg Lys Ser Glu Asp Asp Ser Ala Val Pro Leu
        595                 600                 605
Ala Lys Ala Ala Pro Lys Ser Gly Pro Ser Val Pro Val Ser Val Gln
    610                 615                 620
Thr Lys Asp Asp Val Tyr Glu Ala Phe Met Lys Glu Met Glu Gly Leu
625                 630                 635                 640
Leu

<210> 3
<211> 73
<212> DNA
<213> Artificial Sequence
<220>
<223> Synthesized Oligonucleotide

<400> 3
aattcatggt tccacgtttg ccccctggca gaaaacctcc tggccctccc cctggtccac 60
cttcctcctc aac 73

<210> 4
<211> 68
<212> DNA
<213> Artificial Sequence
<220>
<223> Synthesized Oligonucleotide

<400> 4
gttgaggagg aggtggacca gggggagggc caggaggttt tctgccaggg ggcaaacgtg 60
gaaccatg 68

<210> 5
<211> 29
<212> DNA
<213> Artificial Sequence
<220>

<223> Synthesized Oligonucleotide

<400> 5
gagaattcat gcagatacaa gcacctccc                                29

<210> 6
<211> 27
<212> DNA
<213> Artificial Sequence
<220>
<223> Synthesized Oligonucleotide

<400> 6
gacccgggca atcaagttgg gtggggc                                  27

<210> 7
<211> 26
<212> DNA
<213> Artificial Sequence
<220>
<223> Synthesized Oligonucleotide

<400> 7
gagaattcat gccgctgccc gttgcg                                   26

<210> 8
<211> 27

<212> DNA
<213> Artificial Sequence
<220>
<223> Synthesized Oligonucleotide

<400> 8
gacccgggct tctttgccct cttcccg                                          27

<210> 9
<211> 702
<212> DNA
<213> Homo sapiens

<220>
<221> CDS
<222> (1)..(702)

<400> 9
atg tct cag agc aac cgg gag ctg gtg gtt gac ttt ctc tcc tac aag    48
ctt tcc cag aaa gga tac agc tgg agt cag ttt agt gat gtg gaa gag    96
aac agg act gag gcc cca gaa ggg act gaa tcg gag atg gag acc ccc    144
agt gcc atc aat ggc aac cca tcc tgg cac ctg gca gac agc ccc gcg 192
gtg aat gga gcc act gcg cac agc agc agt ttg gat gcc cgg gag gtg 240
atc ccc atg gca gca gta aag caa gcg ctg agg gag gca ggc gac gag 288
ttt gaa ctg cgg tac cgg cgg gca ttc agt gac ctg aca tcc cag ctc 336
cac atc acc cca ggg aca gca tat cag agc ttt gaa cag gta gtg aat 384
gaa ctc ttc cgg gat ggg gta aac tgg ggt cgc att gtg gcc ttt ttc 432
tcc ttc ggc ggg gca ctg tgc gtg gaa agc gta gac aag gag atg cag 480
gta ttg gtg agt cgg atc gca gct tgg atg gcc act tac ctg aat gac 528
cac cta gag cct tgg atc cag gag aac ggc ggc tgg gat act ttt gtg 576
gaa ctc tat ggg aac aat gca gca gcc gag agc cga aag ggc cag gaa 624
cgc ttc aac cgc tgg ttc ctg acg ggc atg act gtg gcc ggc gtg gtt 672
ctg ctg ggc tca ctc ttc agt cgg aaa tga 702

<210> 10
<211> 31
<212> DNA
<213> Artificial Sequence
<220>
<223> Synthesized Oligonucleotide

<400> 10
gacccgggat atgtctcaga gcaaccggga g 31

<210> 11
<211> 30
<212> DNA
<213> Artificial Sequence
<220>
<223> Synthesized Oligonucleotide

<400> 11
gagcggccgc tcatttccga ctgaagagtg                                        30

<210> 12
<211> 30
<212> DNA
<213> Artificial Sequence
<220>
<223> Synthesized Oligonucleotide

<400> 12
gacacgtgca tccaaactgc tgctgtgcgc                                        30